**Europäisches Patentamt**

**European Patent Office**

**Office européen des brevets**

(11) Veröffentlichungsnummer: **0 371 491**

**A1**

# EUROPÄISCHE PATENTANMELDUNG

(21) Anmeldenummer: 89122037.8

(22) Anmeldetag: 29.11.89

(51) Int. Cl.⁵: **A61L 27/00**

(30) Priorität: 29.11.88 EP 88119847

(43) Veröffentlichungstag der Anmeldung:
06.06.90 Patentblatt 90/23

(84) Benannte Vertragsstaaten:
**AT BE CH DE ES FR GB IT LI NL SE**

(71) Anmelder: **Heinl, Thomas, Dr.**
**Hahlerstrasse 56**
**D-4950 Minden(DE)**

(72) Erfinder: **Heinl, Thomas, Dr.**
**Hahlerstrasse 56**
**D-4950 Minden(DE)**

(74) Vertreter: **Pöhner, Wilfried Anton, Dr.**
**Kaiserstrasse 27 Postfach 63 23**
**D-8700 Würzburg 1(DE)**

(54) **Implantat.**

(57) Das erfindungsgemäße Implantat besteht aus einem metallischen Kernkörper mit mindestens einer Auftragsschicht. Durch die Laserbehandlung bildet sich an der Kontaktfläche zwischen Auftragsschicht und Kernkörper eine Übergangsschicht 24 aus, die eine Dicke von maximal 2µm aufweist. Ferner entsteht durch die Laserbestrahlung ein verdichteter Bereich 23, der sich vorzugsweise bis auf die Übergangsschicht 24 ausdehnt, mindestens aber bis zu den Fortsätzen 25, 26, die die Oberfläche des Kernkörpers 10 im Sinne einer Verklammerung gestalten.

FIG. 1

## Implantat

Die Erfindung betrifft ein Implantat zur Einbringung in Humanknochen mit einem metallischen Kernkörper und mindestens einer darauf aufgebrachten Auftragsschicht sowie Verfahren zur seiner Herstellung.

Aus der DE-OS 33 01 122 ist ein keramischer Knochenersatzwerkstoff bekannt, der aus einem Gemisch von Titanoxid und Hydroxylapatit besteht. Das vorverdichtete Pulver aus 70 - 95 % Titanoxid und 5 - 30 % Ca5(OH) x (PO4)3 führt nach einer Wärmebehandlung bei 1250 - 1450 Grad Celsius zu einer bioaktiven Keramik aus Titandioxid und Trikalziumphosphat. Diese Kombination erbringt zwar eine Festigkeitssteigerung um ca. 10 % gegenüber der monophasischen Hydroxylapatit-Keramik, wird aber auch schneller vom Knochengewebe abgebaut.

· Aus DE-OS 26 59 591 ist es bekannt, auf metallische Kernkörper eine biostabile Primärschicht aus Oxidkeramik und darauf eine biostabile Sekundärschicht aus einer Kalziumphosphatkeramik durch Aufdampfen aufzubringen. Diese Mehrschichtenkeramik kann als Implantat im Bereich lastaufnehmender Strukturen eingesetzt werden.

Feingewebliche Untersuchungen im eigenen Labor an autopsierten Implantat-Knochen-Präparaten führten zu der Erkenntnis, daß die aufgebrachten Schichten aus Hydroxylapatitkeramik von dem sich neu bil denden Knochengewebe durch Lösungs- und zelluläre Prozesse unterwandert werden. Dadurch kommt es nach unseren Beobachtungen zum biomedizinisch unerwünschten Kontakt zum metallischen Grundkörper.

Bei langfristiger Einwirkung können als Folge Ablösungsprozesse der Auftragsschichten auftreten, wodurch es zu einer Lockerung und anschließenden Zerstörung des Verbundes zwischen Implantat und Knochensubstanz kommt.

Aufgabe der vorliegenden Erfindung ist es, ein Implantat mit einer für die biologische Beanspruchung geeigneten Oberfläche so auszurüsten, daß unter Wechselbeanspruchung eine absolute Sperrwirkung zwischen Kernmaterial und bioaktiver Oberflächenschicht bestehen bleibt.

Dies wird durch die in den Patentansprüchen angegebenen Merkmale gelöst.

Der Erfindung liegt die Erkenntnis zugrunde, daß die Porosität der Auftragsschicht zwar eine sehr gute Voraussetzung für das zur mechanischen Festigkeit führende Einwachsen des neu entstehenden Knochengewebes bietet. Aber langfristig besteht die Gefahr der Schichtzerstörung infolge kontinuierlicher Wachstumsprozesse des Knochengewebes in die Tiefe der Schicht selbst bei niedrig belasteten Implantaten.

Dies liegt - wie aufgrund eingehender Studien gefunden - daran, daß im Schichtmaterial im Bereich der Poren und entlang der feinsten Mikrorisse An- und Auflösungsvorgänge auftreten. Es wird daher vorgeschlagen, die Poren und Risse in der Übergangsschicht zum Kernmaterial hin zu verschließen, vor zugsweise die Auftragsschicht zum Kernmaterial hin gezielt zu verdichten.

Die Undurchlässigkeit der Grenzfläche bewirkt, daß die Ionen des metallischen Kernkörpers nicht ins Körperinnere gelangen können, wo sie Anlaß zu Vergiftungen oder allergischen Reaktionen geben können. Aber auch Körpersäfte, unter denen im Sinne der Erfindung alle körpereigenen Flüssigkeiten zu verstehen sind, können andererseits nicht an den metallischen Kernkörper gelangen und dort Korrosionen auslösen. Entscheidend ist, daß keine Permeabilität hinsichtlich der Metallionen sowie der Körpersäfte gegeben ist.

Um das Einwachsen des Humanknochens in die Grenzfläche des Implantates zu erleichtern, sollte die Auftragsschicht nach außen durchlässig sein, d.h. eine hohe Permeabilität aufweisen. Die Auftragsschicht weist im Idealfall einen Gradienten der Durchlässigkeit/Permeabilität, beispielsweise realisiert über die Dichte, auf.

Auch das Vergrößern der Oberfläche der Auftragsschicht, wie sie durch nach außen offene Poren oder durch aufgerauhte Oberflächen erzeugt wird, erleichtert das Anwachsen des Knochens beträchtlich.

Als Verfahren zur Herstellung eines derartigen Implantates wird vorgeschlagen, nach dem Aufbringen der Auftragsschicht deren Verdichtung im Übergangsbereich zum Kernkörper hin dadurch vorzunehmen, daß diese Zone mit energiereichen Strahlen beaufschlagt wird. Die Erwärmung erfolgt hierbei soweit, daß eine Ionendiffusion entsteht, die zur Folge hat, daß eine innige Vereinigung zwischen Kernkörper und Auftragsschicht nach Art einer Diffusionsverbindung entsteht. Die Folge ist eine hohe Haftfestigkeit sowie die Ausbildung einer undurchlässigen Grenzfläche aufgrund der Schichtenverdichtung. Die Energiezufuhr muß einerseits so hoch sein, daß Ionendiffusion sich ausbildet, jedoch andererseits keine Phasenumwandlung des Metalls entsteht. Gegenüber adhäsiver Haftung der Auftragsschicht weist die Diffusionszone eine höhere Haftfestigkeit und Undurchlässigkeit auf.

Als energiereiche Strahlen kommen Laser und Elektronenstrahlen in Betracht, wobei letztere den Vorteil der exakteren Dosierung und Zufuhr von Energie in die Grenzfläche gestattet.

In der Praxis kann dies durch Einwirkung mit einem Laser, durch Heißverdichtung oder mit sonstigen hitzeentwickelnden Einrichtungen vorgenommen werden, deren Schmelzzone lokal begrenzt ist. Es muß dabei sichergestellt werden, daß die zu verdichtenden Primärschichten anschmelzen und gleichzeitig die Wärme in ausreichendem Maße abgeführt werden kann, um zu vermeiden, daß hohe Eigenspannungen und damit Verspannungen bzw. Rißbildungen entstehen.

Im folgenden wird ein Beispiel anhand eines zweifach mit Hydroxylapatit beschichteten Implantats gegeben.

Auf den Kernkörper 10 aus TiAl6V4 wird zunächst eine primäre Keramikschicht 21 aus Ca5-(OH) (PO4)3 aufgetragen. Nach der thermischen Einwirkung mit einem CO2-Laser weist die Keramikschicht 21 einen verdichteten Bereich 23 mit einer Porosität von 0 bis 5 % auf. Diese erhält einen Überzug aus einer unver dichteten Kalziumapatit-Schicht mit 10 bis 30 % Porosität. Die Porosität wird morphometrisch anhand von Querschliffen von Probekörpern bestimmt.

An den aus einem Zeitraum von acht Jahren ausgewerteten Proben menschlichen Knochengewebes nach der Implantation von poröser und dichter Hydroxylapatit-Keramik ergibt sich, daß die Verbindung zwischen der Keramik und dem Knochengewebe eine Verwachsung darstellt, dh. von substantieller Natur (Stoffschluß) ist.

Mit der porösen Hydroxylapatit-Keramik erfolgt das Verwachsen deutlich schneller als mit der dichten Hydroxylapatit-Keramik. Dabei stellen Oberflächenrauhigkeit und Mikroporosität die das Wachstum beschleunigenden Faktoren dar. Der biologisch positive Effekt des schnellen Knochenwachstums bewirkt andererseits, daß die Zellfortsätze nach relativ kurzer Zeit die Metalloberfläche erreichen, woraufhin Ablösungen der Hydroxylapatit-Keramik auftreten.

Im Falle einer Auftragsschicht 20 bestehend aus einer verdichteten Primärschicht 21 und einer bioaktiven Sekundärschicht 22 endet das Fortschreiten des Zellwachstums mit dem Erreichen der Primärschicht. Es wurde gefunden, daß der Hydroxylapatit seine bioaktiven Eigenschaften bei einer mittleren Rauhigkeit von 24 - 45$\mu$m, einer Porosität von 15 % und einer Porengröße von 2 - 5$\mu$m besonders entfaltet. Unter diesen Bedingungen ist die erfindungsgemäße Sekundärschicht knochenzell-wachstumsfördernd. Die Primärschicht 21 aus vorzugsweise Kalziumapatit wird mit einem Laser bestrahlt, der 10 - 20 % außerhalb der Focusebene arbeitet. Dadurch wird eine Spurbreite auf der Primärschichtoberfläche von 7 - 8 mm erzeugt. Anstelle der Primärschicht aus Kalziumapatit können auch andere keramische Materialien wie Titandioxid, Zirkondioxid oder Aluminiumoxid verwendet werden. In diesem Fall muß die Verdichtung mit veränderten Parametern vorgenommen werden, wobei je nach Temperaturleitfähigkeit der verwendeten Keramik die flächenbezogene Leistung und die Vorschubgeschwindigkeit so angepaßt werden müssen, daß ein örtlich begrenztes Aufschmelzen der zu verdichtenden Keramikschicht erfolgt.

Die Verklammerung zwischen metallischem Kernkörper 10 und Primärschicht 21 wird durch die örtlich angeschmolzene Übergangsschicht verbessert, wobei angeschmolzenes, metallisches Material als Erhebungen in die natürlichen Lücken und Hohlräume der Primärschicht eindringt. Zusätzlich kann eine Ionen-Konvektion einen festigkeitssteigernden Effekt bewirken.

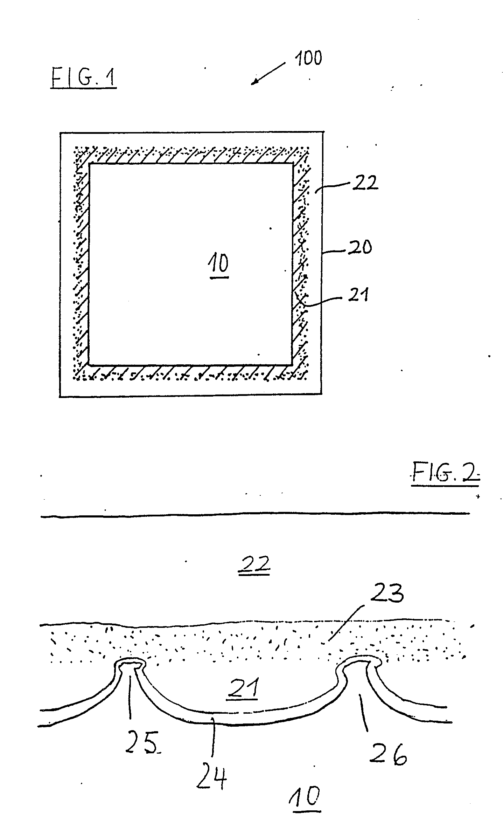

Im folgenden wird anhand von Fig. 2 die Verklammerung der Auftragsschicht mit dem Kernkörper erläutert.

In Fig. 2 ist der metallische Kernkörper 10 und die Primärschicht 21 als vergrößerter Ausschnitt aus Fig. 1 dargestellt. Zwischen beiden Schichten hat sich eine metallionenhaltige Übergangsschicht 24 durch Konvektion während der Wärmebehandlung gebildet. Die Fortsätze 25, 26 das Kernkörpers 10 reichen bis nahe an die Oberfläche der Primärschicht 21, werden aber durch die verdichtete Schicht 23 vollständig gegenüber der Sekundärschicht 22 abgeschirmt.

Zum Nachweis der Dichtigkeit des erfindungsgemäßen Schichtaufbaus wurde ein Vergleichsversuch in physiologischer Kochsalzlösung zur Bestimmung des Metall ionen-Übertritts durchgeführt. Die Gesamtversuchsdauer betrug 12 Monate. Zunächst wurde ein Kernkörper aus TiAl6V4 gegen einen mit poröser Hydroxylapatit-Keramik beschichteten, gleich zusammengesetzten Kernkörper untersucht. Es wurde festgestellt, daß der Metallionen-Übertritt bei dem keramisch beschichteten Kernkörper im Vergleich zum unbeschichteten Körper um den Faktor 40 sinkt.

Unter sonst gleichen Bedingungen wurde ein erfindungsgemäß entsprechend der Anordnung nach Fig. 2 aufgebauter Schichtkörper mit einer verdichteten Primärschicht aus Hydroxylapatit untersucht. Es ergab sich, daß der Metallionen-Übertritt unterhalb der Nachweisgrenze von 0,01 mg/l für die beteiligten Metallionen lag.

## Ansprüche

1) Implantat zur Einbringung in Humanknochen mit einem metallischen Kernkörper und einer Auftragsschicht, **dadurch gekennzeichnet,** daß die Auftragsschicht (20) an der Grenzfläche zum Kernkörper für Metallionen und Körpersäfte undurchlässig ist.

2) Implantat nach Anspruch 1, **dadurch gekennzeichnet,** daß die Durchlässigkeit der Auftragsschicht nach außen zunimmt.

3) Implantat nach Anspruch 1 oder 2, **gekennzeichnet durch** maximale Oberfläche der Auftragsschicht (20).

4. Implantat nach Anspruch 3, **gekennzeichnet durch** nach außen offene Poren.

5) Implantat nach Anspruch 3 oder 4, **gekennzeichnet durch** eine aufgerauhte Oberfläche.

6) Implantat nach einem der Ansprüche 1 bis 5, **dadurch gekennzeichnet,** daß die Auftragsschicht (20) aus einer Primärschicht (21) und einer bioaktiven Sekundärschicht (22) besteht, wobei die Primärschicht (22) eine erhöhte Dichte aufweist.

7) Implantat nach Anspruch 6, **dadurch gekennzeichnet,** daß die Auftragsschicht (20) eine Dicke von 50-200μm und die Primärschicht eine Dicke von 5-50μm aufweist, wobei das Dickenverhältnis von Primärschicht zur gesamten Auftragsschicht (20) 1:2 bis 1:3 beträgt.

8) Implantat nach einem der vorhergehenden Ansprüche, **dadurch gekennzeichnet,** daß mindestens eine der Auftragsschichten aus Kalzium-Phosphat-Keramik besteht.

9) Implantat nach einem der vorhergehenden Ansprüche, **dadurch gekennzeichnet,** daß die Primärschicht (21) aus Oxidkeramik und die Sekundärschicht (22) aus Kalzium-Phosphat-Keramik besteht.

10) Implantat nach einem der vorhergehenden Ansprüche, **dadurch gekennzeichnet,** daß die Primärschicht (21) aus Hydroxylapatit, Titandioxid, Aluminiumoxid oder Zirkondioxid besteht.

11) Implantat nach einem der vorhergehenden Ansprüche, **dadurch gekennzeichnet,** daß der Kernkörper (10) aus einer Titan-, Niob-, Tantal-, Kobaltlegierung oder aus Edelstahl besteht.

12) Implantat nach einem der vorhergehenden Ansprüche, **dadurch gekennzeichnet,** daß der Kernkörper (10) aus einer Titanlegierung, die Primärschicht (21) aus Hydroxylapatit mit einer Porosität von 0 - 5 % und die Sekundärschicht aus Hydroxylapatit mit einer Porosität von 10 - 30 % besteht.

13) Implantat nach einem der vorhergehenden Ansprüche, **dadurch gekennzeichnet,** daß die Kalzium-Phosphat-Keramik aus Kalzium-Triphosphat, Kalzium-Tetraphosphat oder Kalziumphosphatmischungen besteht und daß diese Keramik bis zu 5 % Bestandteile aus Nicht-Kalziumphosphaten aufweist.

14) Implantat nach einem der vorhergehenden Ansprüche, **dadurch gekennzeichnet,** daß die Auftragsschicht (20), die Primärschicht (21) und/oder die Sekundärschicht (22) plasmagespritzt ist.

15) Verfahren zur Herstellung eines Implantats, bestehend aus einem metallischen Kernkörper und mindestens einer Auftragsschicht, **dadurch ge-** kennzeichnet, daß auf dem metallischen Kernkörper (10) eine Primärschicht (21) aufgebracht wird, die Primärschicht durch thermische Behandlung verdichtet und gleichzeitig mit dem metallischen Kernkörper (10) verklammert wird und danach unter Umständen eine Sekundärschicht (22) aus bioaktiver Keramik auf die Primärschicht (21) aufgetragen und mit dieser verbunden wird.

16) Verfahren nach einem der vorhergehenden Ansprüche, **dadurch gekennzeichnet,** daß die Primärschicht (21) mit einer focussierbaren Wärmequelle bei Temperaturen von mehr als 650 Grad Celsius behandelt wird, wobei die örtliche Einwirkungsdauer zwischen 0,02 - 2 Sekunden beträgt.

17) Verfahren nach einem der vorhergehenden Ansprüche, **dadurch gekennzeichnet,** daß die Primärschicht (21), die Sekundärschicht (22) und ggfs. weitere Auftragsschichten durch Plasmaspritzen auf den Kernkörper (10) aufgebracht werden.

18) Verfahren nach einem der vorhergehenden Ansprüche, **dadurch gekennzeichnet,** daß während der Wärmebehandlung Metallionen auf dem Kernkörper(10) in die Grenzzone zwischen Kernkörper (10) und Primärschicht (21) übertreten.

19) Verfahren zur Herstellung eines Implantates nach einem der vorhergehenden Ansprüche, **dadurch gekennzeichnet,** daß die Übergangszone zwischen Kernkörper (10) und Auftragsschicht (20) durch energiereiche Strahlen, wie Laser oder Elektronenstrahl, bis zur Ionendiffusion erwärmt wird.

20) Verfahren nach einem der vorhergehenden Ansprüche, **dadurch gekennzeichnet,** daß die Wärmebehandlung mit einem $CO_2$-Laser unter Schutzgas vorgenommen wird, wobei die Primärschicht (21) außerhalb des Focusbereiches der Laserstrahlen liegt.

21) Verfahren nach einem der vorhergehenden Ansprüche, **dadurch gekennzeichnet,** daß die Primärschicht (21) 100 - 200 mm außerhalb des Focusbereiches des $CO_2$-Lasers liegt.

22) Verfahren nach einem der vorhergehenden Ansprüche, **dadurch gekennzeichnet,** daß der Laser im CW-Betrieb mit einer Vorschubgeschwindigkeit von 0,1 - 20 m pro Minute über den primärbeschichteten Kernkörper (10) geführt wird.

FIG. 1

100

10

22

20

21

FIG. 2

22

23

21

25

24

26

10

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 606 617 (PLASMAINVENT AG) <br> * Ansprüche; Figur 1; Seite 4, Zeilen 19-24; Seite 5, Zeilen 14-20; Seite 6, Zeilen 16-27; Seite 7, Zeilen 7-26 * | 1-11,14 ,17 | A 61 L 27/00 |
| Y | --- | 13 | |
| Y | DE-A-3 709 457 (PERMELEC ELECTRODE LTD) <br> * Seite 3, Zeile 46 - Seite 4, Zeile 56 * | 13 | |
| A | DE-A-2 936 513 (THE UNIVERSITY OF UTAH) <br> * Seite 8, Zeilen 6-14; Seite 5, Zeilen 6-15 * | 19-22 | |
| A | WO-A-8 603 977 (BATELLE INSTITUT) <br> * Seite 4, Zeilen 8-16 * | 15-16 | |
| A,D | DE-A-2 659 591 (SUMITOMO) <br> --- | | |
| A,D | DE-A-3 301 122 (J.F. OSBORN) <br> --- | | RECHERCHIERTE SACHGEBIETE (Int. Cl.5) |
| A | US-A-4 542 539 (R. ROWE) <br> * Ansprüche; Spalte 3, Zeilen 26-39; Spalte 4, Zeilen 46-68; Figuren 1-4 * | | A 61 L <br> A 61 K |

Der vorliegende Recherchenbericht wurde für alle Patentansprüche erstellt

| Recherchenort | Abschlußdatum der Recherche | Prüfer |
|---|---|---|
| DEN HAAG | 02-03-1990 | COUSINS-VAN STEEN G.I.L. |